# EUROPEAN PATENT SPECIFICATION

(11) **EP 1 313 462 B1**
(45) Date of publication and mention of the grant of the patent: **03.12.2008**
(21) Application number: 01920974.1
(22) Date of filing: 14.02.2001
(51) Int. Cl.: A61K 31/19, A61K 31/704, A61K 45/06, A61P 35/00

(54) **USE OF ASIATIC ACID OR ASIATICOSIDE FOR TREATMENT OF CANCER**
VERWENDUNG VON ASIATSÄURE ODER ASIATICOSID ZUR BEHANDLUNG VON KREBS
UTILISATION D'ACIDE ASIATIQUE OU D'ASIATICOSIDE POUR LE TRAITEMENT DU CANCER

(30) Priority: 29.08.2000 MY 0003987
(43) Date of publication of application: 28.05.2003
(73) Proprietor: Government of Malaysia, 62662 Putrajaya (MY); MASSACHUSETTS INSTITUTE OF TECHNOLOGY, Cambridge, MA 02139 (US)
(72) Inventor: OH, Se-Kyung Massachusetts Institute of Technology, Cambridge, MA 02139 (US); RHA, ChoKyun Massachusetts Institute of Technology, Cambridge, MA 02139 (US); ABDUL KADIR Azizol, Kuala Lumpur (MY); NG, Lean Teik, Kuala Lumpur (MY)
(74) Representative: Murphy, Colm Damien
(86) International application number: PCT/US2001/040103
(87) International publication number: WO 2002/017904

(56) References cited:
- EP-A- 0 383 171
- EP-A- 0 852 946
- WO-A-20/05120495
- GB-A- 923 414
- VOGEL H G ET AL: "EFFECT OF TERPENOIDS ISOLATED FROM CENTELLA-ASIATICA ON GRANULOMA TISSUE" ACTA THERAPEUTICA, vol. 16, no. 4, 1990, pages 285-298, XP001030891 ISSN: 0378-0619
- BADER, G. ET AL: "Cytotoxicity of triterpenoid saponins. Part 1. Activities against tumor cells in vitro and hemolytic index" PHARMAZIE (1996), 51(6), 414-417 , XP001024624
- PLOHMANN B ET AL: "Immunomodulatory effects of triterpenoid saponins." EUROPEAN JOURNAL OF PHARMACEUTICAL SCIENCES, vol. 2, no. 1-2, 1994, page 120 XP001030425 Second European Congress of Pharmaceutical Sciences;Berlin, Germany; September 29-October 1, 1994 ISSN: 0928-0987
- BONNETT G.F.: "[Treatment of localized cellulitis with asiaticoside Madecassol ]. TRAITEMENT DES CELLULITES LOCALISEES PAR LE MADECASSOL." PROGRES MEDICAL, (1974) 102/3 (109-110). CODEN: PRMIAU, XP001030401
- DATABASE CA [Online] CHEMICAL ABSTRACTS SERVICE, COLUMBUS, OHIO, US; QI, SHAOHAI ET AL: "Effects of asiaticoside on hypertrophic scars in a nude mice model" retrieved from STN Database accession no. 133:344288 XP002180967 & ZHONGHUA SHAOSHANG ZAZHI (2000), 16(1), 53-56 ,
- PATENT ABSTRACTS OF JAPAN vol. 1996, no. 09, 30 September 1996 (1996-09-30) & JP 08 133952 A (SHISEIDO CO LTD), 28 May 1996 (1996-05-28)
- RUSH W.R. ET AL: "The comparative steady-state bioavailability of the active ingredients of Madecassol." EUROPEAN JOURNAL OF DRUG METABOLISM AND PHARMACOKINETICS, (1993) 18/4 (323-326). , XP001029982 cited in the application
- DERAMOUDT B. ET AL: "[The cellulodystrophic syndrome]. LE SYNDROME CELLULODYSTROPHIQUE." ANNALES DE READAPTATION ET DE MEDECINE PHYSIQUE, (1987) 30/4 (433-438). , XP001030400
- PISHA E ET AL: "DISCOVERY OF BETULINIC ACID AS A SELECTIVE INHIBITOR OF HUMAN MELANOMA THAT FUNCTIONS BY INDUCTION OF APOPTOSIS" NATURE MEDICINE, NATURE PUBLISHING, CO, US, vol. 1, no. 10, 1 October 1995 (1995-10-01), pages 1046-1051, XP000611705 ISSN: 1078-8956
- BABU T D ET AL: "Cytotoxic and anti-tumour properties of certain taxa of Umbelliferae with special reference to Centella asiatica (L.) Urban." JOURNAL OF ETHNOPHARMACOLOGY, vol. 48, no. 1, 1995, pages 53-57, XP001030036 ISSN: 0378-8741 cited in the application
- SKALETZ-ROROWSKI A ET AL: "Asiatic acid and asiaticoside of Centella asiatica inhibit the proliferation of coronary smooth muscle cells." PHYTOMEDICINE (JENA), vol. 7, no. Supplement 2, 2000, page 114 XP001032453 3rd International Congress on Phytomedicine;Munich, Germany; October 11-13, 2000 ISSN: 0944-7113
- SAMPSON J.H. ET AL: "In vitro keratinocyte antiproliferant effect of Centella asiatica extract and triterpenoid saponins." PHYTOMEDICINE, (2001) 8/3 (230-235). , XP001030446
- G. W. FRANK: "The fascination of Kombucha" THE AMERICAN RAUM & ZEIT, vol. 2, no. 5, 1991, pages 51-56,

## Description

### Field of the Invention

The invention relates to the use of asiatic acid and precursor compounds for the treatment of diseases involving cell proliferation, and in particular cancer, as specified in claim 1.

### Background of the Invention

Asiatic acid is a component of the medicinal plant *Centella asiatica,* commonly known as Gotu Kola or Indian pennywort. *Centella asiatica* is a creeping plant indigenous to the tropical and swampy regions of, among others, central Asia, Madagascar and South Africa. Traditionally the roots and leaves of *Centella asiatica* have been used medicinally to treat various skin conditions ranging from slow healing wounds and lesions, to leprosy. Additional traditional uses of *Centella asiatica* include heart disease, high blood pressure, rheumatism, fevers, nervous disorders, bronchitis, asthma and syphylis. (Duke, J.A. CRC Handbook of medicinal herbs. Boca Raton, FL: CRC Press, 1985, 110-111).

The active compounds of *Centella asiatica* were first isolated in the early 1940's. (J.E. Bontems, Bull. Sci. Pharmacol., (1941) 49, 186-96). The active ingredients of *Centella asiatica* were determined to be triterpenoids (also called saponins) the constituents of which include: asiaticoside, madecassoside, madasiatic acid and asiatic acid. Topical application of extracts purified from *Centella asiatica* have been shown to aid in wound healing, burns, chronic venous insufficiency and the treatment and prevention of enlarged scar tissue (keloids). (Kartnig T., In Herbs, Spices, and Medicinal Plants: Recent Advances in Botany, Horticulture, and Pharmacology, vol. 3., ed LE Craker, JE Simon. Phoenix, AZ: Oryx Press, 1986, 145-73; Mahajani SS, et al., Can J Physiol Pharmacol 1994;72 (suppl 1):180; Pointel JP, et al., Angiology 1986; 37(5) :420-21. Bosse JP, et al., Ann Plastic Surg 1979; 3:13-21). Isolated extracts may promote wound healing through the activation of malpighean cells and the induction of keratinization. (May, A. Eur. J. Pharmacol., (1968) 4(3), 331-9).

Presently, extracts from *Centella asiatica* are commonly used as the active ingredients of many cosmetic preparations in the skin care industry. Asiatic acid and/or asiaticoside are used as the active ingredients in dermatological compositions for the treatment of hair and skin. The asiatic and madecassic acids are additionally known to cause collagen production, which endows the compounds with wound healing and anti-cellulite properties. Additionally asiaticoside has been used clinically in the treatment of systemic scleroderma. (Sasaki, S., et al. Acta Diabetol La (1972) 52:141-50). Medecassol, a titrated extract of *Centella asiatica,* is a commercially available oral therapeutic for the treatment of keloids, the proliferation of connective tissue, and hypertrophic scars.

Asiatic acid is reported to be the active ingredient of Medecassol. (Rush WR, et al., Eur J Drug Metab Pharmacokinet., (1993) 18 (4) :323-6). Asiatic acid derivatives have been further shown to independently function as wound healing agents and protective agents against beta-amyloid induced neurotoxicity. (U.S. patent 5,834,437; Mook-Jung, I., et al., J Neurosci. Res., (1999) 58 (3) :417-25).

Asiatic acid has the following chemical structure:

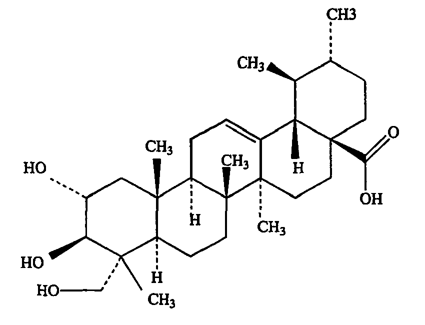

Asiaticoside is a glycoside precursor of asiatic acid. Asiaticoside is converted *in vivo* to asiatic acid by hydrolytic cleavage of the sugar moiety, presumably by the action of glycosidases. (Rush WR, et al., Eur JDrug Metab Pharmacokinet., (1993) 18(4): 323-6). The therapeutic effects of asiaticoside are postulated to be mediated through conversion to asiatic acid, as the sugar moiety does not appear to be required for at least certain biological activities. (Bonte, F., et al, Planta Med., (1994) 60(2):133-5).

U.S. patent 6,071,898 discloses asiatic acid analogs and their use for the treatment of diseases including cancer. The 6,071,898 patent does not disclose the use of asiatic acid or natural precursors such as asiaticoside. The asiatic acid derivatives disclosed in U.S. patent 6,071,898 exhibit severe structural modifications to the asiatic acid compound. The synthesized derivatives have a modified A-ring where the hexameric ring found in asiatic acid is modified to a pentameric configuration. The asiatic acid derivatives are further modified by the addition of two bulky hydrocarbon groups attached to the A-ring. The modifications are not minor and result in a chemical compound that is appreciably altered from asiatic acid.

Partially purified fractions of *Centella asiatica* have been shown to exhibit anti-tumour effects both in vitro, using transformed cell lines, and in vivo using model systems. However, although experimental data suggests that the effect is mediated via an alteration in DNA synthesis, the active compound was not identified by the investigators. (Babu, T.D., et al., J. Ethnopharmacol. (1995) 48(1): 53-7).

The cytoxicity of triterperoid saporins in the context of tumour cells in vitro is discussed by Bader et al., in Pharmazie (1996) 51(6), 414-417.

Many compounds are used for the treatment of cancer and other diseases involving cellular proliferation. Many proliferative diseases, including cancer, remain difficult to treat due to the side effects of presently available treatments, the lack of effective treatments, etc. Accordingly, new compositions for treating proliferative diseases, particularly the many types of cancer, are needed.

### Summary of the Invention

The invention is based on the discovery that asiatic acid is cytotoxic to cancer cells and induces apoptosis in these cells. It was unexpected that asiatic acid would be cytotoxic in view of its prior use in topical formulations for many cosmetic preparations in the skin care industry, wound healing, anti-cellulite treatments, systemic scleroderma, the treatment of keloids, and hypertrophic scars.

According to the present invention, there is provided use of a composition comprising an isolated molecule of asiatic acid or asiaticoside in the manufacture of a medicament for use in the treatment of cancer of epithelial cell origin. The amount of the composition is effective to treat the cancer. In certain embodiments, the isolated molecule is asiatic acid. In other embodiments, the isolated molecule is asiaticoside. In preferred embodiments, the treatment inhibits further growth of cancer or results in regression of cancer. In certain preferred embodiments, the subject is otherwise free of symptoms treatable by asiatic acid or asiaticoside. In other preferred embodiments, the treatment further includes administering to the subject an anticancer compound other than asiatic acid or asiaticoside.

Also described is a composition for use in inhibiting cell proliferation. The use includes contacting a cell with an amount of a composition comprising an isolated asiatic acid molecule or asiaticoside molecule, in an amount effective to inhibit the proliferation of the cell. In certain embodiments, the isolated molecule is asiatic acid. In other embodiments, the isolated molecule is asiaticoside.

Also described is the use of a composition for increasing apoptosis in a cell or population of cells. The use includes contacting the cell or population of cells with an amount of a composition including an isolated molecule selected from the group consisting of asiatic acid and asiaticoside, effective to increase apoptosis. In certain embodiments, the isolated molecule is asiatic acid. In other embodiments, the isolated molecule is asiaticoside.

Further described are pharmaceutical preparations. The pharmaceutical preparations include an amount of an isolated molecule selected from the group consisting of asiatic acid and asiaticoside effective to treat cancer, and a pharmaceutically acceptable carrier. In certain embodiments, the isolated molecule is asiatic acid. In other embodiments, the isolated molecule is asiaticoside. In still other embodiments, the pharmaceutical preparations include an anticancer compound which is not asiatic acid or asiaticoside.

Additionally described are products which include an anticancer effective amount of an isolated molecule selected from the group consisting of asiatic acid and asiaticoside in a unit dosage, and instructions for administration of the isolated molecule.

These and other aspects of the invention will be described in greater detail below.

### Detailed Description of the Invention

The compounds useful in the invention are asiatic acid and asiaticoside, which have the following structures:
Asiatic acid: 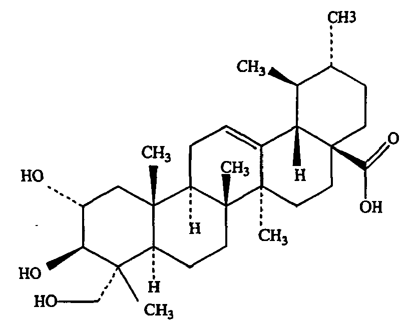
Asiaticoside:

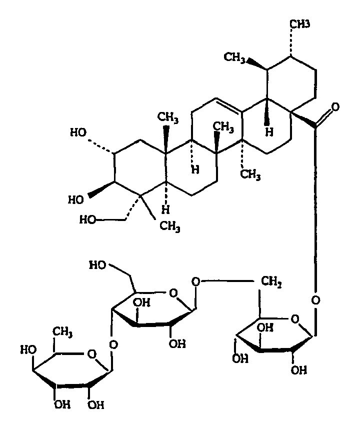

Asiatic acid and asiaticoside are used for treating subjects who have conditions involving proliferation, i.e., proliferative diseases. Proliferative diseases include cancer, psoriasis, lipoma, adenomas from any organ tissue (i.e. colon polyps), and polycystic kidney disease. The proliferative conditions described above typically are not associated with skin conditions such as wounds, cellulite, systemic scleroderma, keloids and hypertrophic scars.

Cancer, as used herein, includes the following types of cancer, breast cancer, biliary tract cancer; bladder cancer; brain cancer including glioblastomas and medulloblastomas; cervical cancer; choriocarcinoma; colon cancer; endometrial cancer; esophageal cancer; gastric cancer; hematological neoplasms including acute lymphocytic and myelogenous leukemia; multiple myeloma; AIDS-associated leukemias and adult T-cell leukemia lymphoma; intraepithelial neoplasms including Bowen's disease and Paget's disease; liver cancer; lung cancer; lymphomas including Hodgkin's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcoma, and osteosarcoma; skin cancer including melanoma, Kaposi's sarcoma, basocellular cancer, and squamous cell cancer; testicular cancer including germinal tumors such as seminoma, non-seminoma (teratomas, choriocarcinomas), stromal tumors, and germ cell tumors; thyroid cancer including thyroid adenocarcinoma and medullar carcinoma; and renal cancer including adenocarcinoma and Wilms tumor. Other cancers will be known to one of ordinary skill in the art and also can be treated using compositions including asiatic acid and/or asiaticoside. .

Asiatic acid and asiaticoside also can be used to inhibit the proliferation of cells by the induction of apoptosis. Apoptosis refers to the process of programmed cell death. Apoptosis guides cell selection and regulation of cell population in the developing organism. In a mature organism, apoptosis additionally functions to rid the body of damaged or mutated cells. Cancerous cells which exhibit abnormal proliferation are thought to lack the ability to undergo appropriate apoptotic cell death. The process of apoptosis differs from simple necrosis which is a non-programmed form of cell death in response to injury. Apoptotic cell death is partially triggered by the activation of certain genes, such as transcription factors. A gene common to many human cancers, p53, is capable, in its unmutated form, of inducing apoptosis and halting cellular proliferation.

Apoptosis can be measured by standard assays well known to those of skill in the art. Such assays include analysis of DNA ladder formation, TDT-mediated dUTP-biotin nick end labeling (TUNNEL), cell morphology, etc.

Asiatic acid and asiaticoside are commercially available compounds, are derived from commercially available compounds or are synthesized *de novo* using routine chemical synthetic procedures known to those of ordinary skill in the art. Asiatic acid and asiaticoside may be obtained in their purified form from MMP, Inc. (3470 South Clinton Ave., South Plainfield, NJ 07080), suppliers of raw materials to both the health care and the cosmetic industries.

Also contemplated is the use of asiatic acid and asiaticoside in experimental model systems to determine the role that cell proliferation plays in a variety of conditions. Cell proliferation can be induced experimentally, and then asiatic acid and asiaticoside as described above is administered to the animal or contacted with the cell culture being tested. The application may be local or may be systemic. The response or the animal or cell culture is monitored and compared to control animals or cell cultures that do not receive the asiatic acid and asiaticoside.

Several tests can be used to confirm the antiproliferative activity of asiatic acid or asiaticoside. For example, the tests of cytotoxicity described in the Examples may be used with additional tumor cell lines and tissue samples in cell culture. The assays include *in vitro* cell growth assays including assays of monolayer growth and growth in soft agar and *in vivo* assays of tumor growth.

The invention is particularly directed to a patient population neyer before treated with drugs useful according to the invention, including patients who are not suffering from a disorder such as wounds, cellulite, systemic scleroderma, keloids, and hypertrophic scars. In other words, the treatment preferably is directed to patient populations that otherwise are free of symptoms that call for treatment with any of the drugs useful according to the invention.

When administered, the formulations of the invention are applied in pharmaceutically acceptable amounts and in pharmaceutically acceptable compositions. Such preparations may routinely contain salts, buffering agents, preservative, compatible carriers, and optionally other therapeutic ingredients. Such pharmacologically and pharmaceutically acceptable salts include those prepared from the following acids: hydrochloric, hydrobromic, sulphuric, nitric, phosphoric, maleic, acetic, salicylic, p-toluene sulfonic, tartaric, citric, methane sulfonic, formic, malonic, succinic, naphthalene-2-sulfonic, and benzene sulfonic. Also, pharmaceutically acceptable salts can be prepared as alkaline metal or alkaline earth salts, such as sodium, potassium or calcium salts of the carboxylic acid group.

The compounds useful in the invention may be delivered in a mixture with other anti-proliferative agents (particularly anti-cancer agents) which are not asiatic acid or asiaticoside. One of ordinary skill in the art is familiar with a variety of anti-proliferative agents which are used in the medical arts to treat proliferative diseases such as cancer. Such agents include the following sub-classes of compounds: Antineoplastic agents such as: Acivicin; Aclarubicin; Acodazole Hydrochloride; Acronine; Adozelesin; Adriamycin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Buniodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Caracemide; Carbetimer; Carboplatin; Carmustine; Carubicin Hydrochloride; Carzelesin; Cedefingol; Chlorombucil; Cirolemycin; Cisplatin; Cladribine; Crisnatol Mesylate; Cyclophosphamide; Cytarabine; Dacarbazine; DACA (N-[2- (Dimethyl-amino)ethyl]acridine-4-carboxamide); Dactinomycin; Daunorubicin Hydrochloride; Daunomycin; Decitabine; Dexormaplatin; Dezaguanine; Dezaguanine Ifesylate; Diaziquone; Docetaxel; Doxorubicin; Doxorubicin Hydrochloride; Droloxifene; Droloxifene Citrate; Dromostanolone Propionate; Duazomycin; Edatrexate; Eflornithine Hydrochloride; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Ethiodized Oil I 131; Etoposide; Etoposide Phosphate; Etoprine; Fadrozole Hydrochloride; Fazarabine; Fenretinide; Floxuridine; Fludarabine Phosphate; Fluorouracil; 5-FdUMP; Flurocitabine; Fosquidone; Fostriecin Sodium; Gemcitabine; Gemcitabine Hydrochloride; Gold Au 198; Hydroxyurea; Idarubicin Hydrochloride; Ifosfamide; Ilmofosine; Interferon Alfa-2a; Interferon Alfa-2b; Interferon Alfa-n1; Interferon Alfa-n3; Interferon Beta-1a; Interferon Gamma-1b; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin, Mitosper; Mitotane; Mitoxantrone Hydrochloride; Mycophenolic Acid; Nocodazole; Nogalamycin; Ormaplatin; Oxisuran; Paclitaxel Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rogletimide; Safingol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Strontium Chloride Sr 89; Sulofenur; Talisomycin; Taxane; Taxoid; Tecogalan Sodium; Tegafur; Teloxantrone Hydrochloride; Temoporfin; Teniposide; Teroxirone; Testolactone; Thiamiprine; Thioguanine; Thiotepa; Thymitaq; Tiazofurin; Tirapazamine; Tomudex; TOP-53; Topotecan Hydrochloride; Toremifene Citrate; Trestolone Acetate; Triciribine Phosphate; Trimetrexate; Trimetrexate Glucuronate; Triptorelin; Tubulozole Hydrochloride; Uracil Mustard; Uredepa; Vapreotide; Verteporfin; Vinblastine; Vinblastine Sulfate; Vincristine; Vincristine Sulfate, Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride; 2-Chlorodeoxyadenosine; 2'-Deoxyformycin; 9-aminocamptothecin; raltitrexed; N-propargyl-5, 8-dideazafolic acid, 2-chloro-2'-arabino-fluoro-2'-deoxyadenosine; 2-chloro-2'-deoxyadenosine; anisomycin; trichostatin A; hPRL-G129R; CEP-751; and linomide.

Other anti-neoplastic compounds include; 20-epi-1,25 dihydroxyvitamin D3; 5-ethynyluracil; abiraterone; aclarubicin; acylfulvene; adecypenol; adozelesin; aldesleukin; ALL-TK antogonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives (e.g., 10-hydroxy-camptothecin); canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorins; chloroquinoxaline sulfonamide; cicaprost; cis-porphyrin; cladribine; clomifene analogues; clotrimazole; collismycin A; collismycin 13; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin 10 deslorelin; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; discodermolide; docosanol; dolasetron; doxifluridine; droloxifene; dronabinol; duocarmycin SA; ebselen; ecomustine; edelfosine; edrecolomab; eflornithine; elemene; emitefur; epirubicin; epothilones (A, R = H; B, R = Me); epithilones; epristeride; estramustine analogue; estrogen agonists; estrogen antagonists; etanidazole; etoposide; etoposide 4'-phosphate (etopofos); exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; irinotecan; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide + estrogen + progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mithracin; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A + myobacterium cell wall sk; mopidamol; multiple drug resistance gene inhibitor, multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone + pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; oracin; oral cytokine inducer; ormaplatin; osaterone; oxaliplatin; oxaunomycin; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; podophyllotoxin; porfimer sodium; porfiromycin; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; Sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thalidomide; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene dichloride; topotecan; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; zinostatin stimalamer.

Anti-cancer Supplementary Potentiating Agents: Tricyclic anti-depressant drugs (e.g., imipramine, desipramine, amitryptyline, clomipramine, trimipramine, doxepin, nortriptyline, protriptyline, amoxapine and maprotiline); non-tricyclic anti-depressant drugs (e.g., sertraline, trazodone and citalopram); Ca²⁺ antagonists (e.g., verapamil, nifedipine, nitrendipine and caroverine); Calmodulin inhibitor (e.g. prenylamine, trifluoroperazine and clomipramine); Amphotericin B; Triparanol analogues (e.g. tamoxifen); antiarrhythmic drugs (e.g., quinidine); antihypertensive drugs (e.g. reserpine); Thiol depleters (e.g., buthionine and sulfoximine) and Multiple Drug Resistance reducing agents such as Cremaphor EL. The compounds of the invention also can be administered with cytokines such as granulocyte colony stimulating factor.

Antiproliferative agent : Piritrexim Isethionate. Antiprostatic hypertrophy agent: Sitogluside. Benign prostatic hyperplasia therapy agent: Tamsulosin Hydrochloride. Prostate growth inhibitor: Pentomone.

Radioactive agents : Fibrinogen I 125; Fludeoxyglucose F18; Fluorodopa F 18; Insulin I 125; Insulin I 131; Iobenguane I 123; Iodipamide Sodium I 131; Iodoantipyrine I 131; Iodocholesterol I 131; Iodohippurate Sodium I 123; Iodohippurate Sodium I 125; Iodohippurate Sodium I 131; Iodopyracet I 125; Iodopyracet I 131; Iofetamine Hydrochloride I 123; Iomethin I 125; Iomethin I 131; Iothalamate Sodium I 125; Iothalamate Sodium I 131; Iotyrosine I 131; Liothyronine I 125; Liothyronine I 131; Merisoprol Acetate Hg 197; Merisoprol Acetate- Hg 203; Merisoprol Hg 197; Selenomethionine Se 75; Technetium Tc 99m Atimony Trisulfide Colloid; Technetium Tc 99m Bicisate; Technetium Tc 99m Disofenin; Technetium Tc 99m Etidronate; Technetium Tc 99m Exametazime; Technetium Tc 99m Furifosmin; Technetium Tc 99m Gluceptate; Technetium 99m Lidofenin; Technetium Tc 99mm Mebrofenin; Technetium Tc 99m Medronate; Technetium Tc 99m Medronate Disodium; Technetium Tc 99m Mertiatide; Technetium Tc 99m Oxidronate; Technetium Tc 99m Pentetate; Technetium Ic 99m Pentetate Calcium Trisodium; Technetium Tc 99m Sestamibi; Technetium Tc 99m Siboroxime; Technetium Tc 99m Succimer; Technetium Tc 99m Sulfur Colloid; Technetium Tc 99m Teboroxime; Technetium Tc 99m Tetrofosmin; Technetium Tc 99m Tiatide; Thyroxine I 125: Thyroxine I 131; Tolpovidone I 131; Triolein I 125; Triolein I 131.

Also disclosed are novel compositions of matter that are covalent conjugates for cell targeting agents and asiatic acid or asiaticoside. For example, dehydroascorbic acid (DHA) and other naturally occurring, unbranched fatty acids may be conjugated to asiatic acid or asiaticoside and used according to the methods known in the art to target cancer cells. For example, the methods disclosed in PCT application PCT/US00/06160 may be used to conjugate fatty acids to asiatic acid or asiaticoside. Those of ordinary skill in the art will recognize also numerous other agents for targeting asiatic acid or asiaticoside to particular cells or tissues.

Asiatic acid and/or asiaticoside are administered in effective amounts. An effective amount is a dosage of the asiatic acid or asiaticoside sufficient to provide a medically desirable result. The effective amount will vary with the particular condition being treated, the age and physical condition of the subject being treated, the severity of the condition, the duration of the treatment, the nature of the concurrent therapy (if any), the specific route of administration and like factors within the knowledge and expertise of the health practitioner. For example, an effective amount for treating cancer would be an amount sufficient to lessen or inhibit altogether cancer cell proliferation so as to slow or halt the development of or the progression of a tumor. Preferably, the effective amount is sufficient to decrease the size of a tumor. Thus, it will be understood that asiatic acid or asiaticoside can be used to treat proliferative diseases such as cancer prophylactically in subjects at risk of developing proliferative disease. It is preferred generally that a maximum dose is used; that is, the highest safe dose according to sound medical judgement.

Generally, doses of active compounds will be from 0.01 µg/kg per day to 1000 mg/kg per day. It is expected that doses range of 50-500 µg/kg will be suitable, preferably orally and in one or several administrations per day. Taxol, a leading chemotherapeutic, is administered at the following dosages: for ovarian cancer at 135-175 mg/m² per day every three weeks, and for breast cancer at 175-250 mg/m² per day every three weeks. Doses of asiatic acid or asiaticoside similar to doses of taxol or other chemotherapeutic compounds are contemplated. One of ordinary skill in the art can determine optimal doses using only routine experimentation. Lower doses will result from other forms of administration, such as intravenous administration. In the event that a response in a subject is insufficient at the initial doses applied, higher doses (or effectively higher doses by a different, more localized delivery route) may be employed to the extent that patient tolerance permits. Multiple doses per day are contemplated to achieve appropriate systemic levels of compounds.

When administered, the pharmaceutical preparations of the invention are applied in pharmaceutically-acceptable amounts and in pharmaceutically-acceptably compositions. Such preparations may routinely contain salt, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof. Such pharmacologically and pharmaceutically-acceptable salts include those prepared from the following acids; hydrochloric, hydrobromic, sulfuric, nitric, phosphoric, maleic, acetic, salicylic, citric, formic, malonic and succinic. Also, pharmaceutically-acceptable salts can be prepared as alkaline metal or alkaline earth salts, such as sodium, potassium or calcium salts.

Asiatic acid or asiaticoside may be combined, optionally, with a pharmaceutically-acceptable carrier. The term "pharmaceutically-acceptable carrier" as used herein means one or more compatible solid or liquid filler, diluents or encapsulating substances which are suitable for administration into a human or other animal. The term "carrier" denotes an organic or inorganic ingredient, natural or synthetic, with which the active ingredient is combined to facilitate the application. The components of the pharmaceutical compositions also are capable of being co-mingled with the molecules of the present invention, and with each other, in a manner such that there is no interaction which would substantially, impair the desired pharmaceutical efficacy.

The pharmaceutical compositions may contain suitable buffering agents, including: acetic acid in a salt; citric acid in a salt; boric acid in a salt; and phosphoric acid in a salt.

The pharmaceutical compositions also may contain, optionally, suitable preservatives, such as: benzalkonium chloride; chlorobutanol; parabens and thimerosal.

Compositions suitable for parenteral administration conveniently comprise a sterile aqueous preparation of asiatic acid or asiaticoside, which is preferably isotonic with the blood of the recipient. This aqueous preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1, 3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or di-glycerides. In addition, fatty acids such as oleic acid may be used in the preparation of injectables. Carrier formulation suitable for oral, subcutaneous, intravenous, intramuscular, etc. and their administrations can be found in Remington's Pharmaceutical Sciences, Mack Publishing Co., Easton, PA.

A variety of administration routes are available. The particular mode selected will depend of course, upon the particular drug selected, the severity of the condition being treated and the dosage required for therapeutic efficacy. The invention, generally speaking, may be practiced using any mode of administration that is medically acceptable, meaning any mode that produces effective levels of the active compounds without causing clinically unacceptable adverse effects. Such modes of administration include oral, rectal, topical, nasal, interdermal, or parenteral routes. The term "parenteral" includes subcutaneous, intravenous, intramuscular, intumoral or infusion. Oral administration will be preferred for prophylactic treatment because of the convenience to the patient as well as the dosing schedule.

The pharmaceutical compositions may conveniently be presented in unit dosage form and may be prepared by any of the methods well-known in the art of pharmacy. All methods include the step of bringing asiatic acid or asiaticoside into association with a carrier which constitutes one or more accessory ingredients. In general, the compositions are prepared by uniformly and intimately bringing asiatic acid or asiaticoside into association with a liquid carrier, a finely divided solid carrier, or both, and then, if necessary, shaping the product.

Compositions suitable for oral administration may be presented as discrete units, such as capsules, tablets, lozenges, each containing a predetermined amount of asiatic acid or asiaticoside. Other compositions include suspensions in aqueous liquids or non-aqueous liquids such as a syrup, elixir or an emulsion other delivery systems can include time-release, delayed release or sustained release delivery systems. Such systems can avoid repeated administrations of asiatic acid or asiaticoside, increasing convenience to the subject and the physician. Many types of release delivery systems are available and known to those of ordinary skill in the art. They, include polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Patent 5,075,109. Delivery systems also include non-polymer systems that are: lipids including sterols such as cholesterol, cholesterol esters and fatty acids or neutral fats such as mono di- and tri-glycerides; hydrogel release systems; silastic system; peptide based systems; wax coatings; compressed tablets using conventional binders and excipients; partially fused implants. Specific examples include (a) erosional systems in which asiatic acid or asiaticoside is contained in a form within a matrix such as those described in U.S. Patent Nos. 4,452,775, 4,667,014, 4,748,034 and 5,239,660 and (b) diffusional systems in which an active component permeates at a controlled rate from a polymer such as described in U.S. Patent Nos. 3,832,253, and 3,854,480. In addition, pump-based hardware delivery systems can be used, some of which are adapted for implantation.

Use of a long-term sustained release implant may be particularly suitable for treatment of chronic conditions. Long-term release, are used herein, means that the implant is constructed and arranged to delivery therapeutic levels of the active ingredient for at least 30 days, and preferably 60 days. Long-term sustained release implants are well-known to those of ordinary skill in the art and include some of the release systems described above.

### Examples

### Example 1: Cytotoxicity of Asiatic Acid

Pure asiatic acid was shown to exhibit cytotoxic effect on human foreskin fibroblast cells in culture. Further study has shown that this cytotoxic effect of asiatic acid on foreskin fibroblast cells is dose-dependent. The cytotoxic effects must necessarily result from the application of pure asiatic acid as the purity of the compound was examined by HPLC analysis and no apparent impurities were evident.

The following data quantify the percentage of asiatic acid cytotoxicity as compared with taxol. Taxol is a commercially available chemotherapeutic predominantly used in the treatment of breast cancer, lung cancer and ovarian cancer. The experiments were performed on human foreskin fibroblast cells as well as established tumor cell lines. The percentage cytotoxicity was measured by the MTT dye reduction test, which requires the NADPH dehydrogenase enzyme of the mitochondria in live cells (1).
A) New Born Foreskin Fibroblast cells

| **Conc. (µg/ml)** | **Asiatic Acid (% cytotox)** | **Taxol (% cytotox)** |
|---|---|---|
| 25 | 84 | 55 |
| 12.5 | 80 | 50 |
| 6.25 | 30 | 40 |
| 0.8 | 30 | 40 |
| 0 | 0 | 0 |

B) Squamous carcinoma cells of SCC15

| **Conc. (**µ**g/ml)** | **Asiatic Acid (% cytotox)** | **Taxol (% cytotox)** |
|---|---|---|
| 25 | 90 | 23 |
| 20 | 86 | 8 |
| 10 | 88 | 0 |
| 5 | 20 | 0 |
| 2.5 | 16 | 3 |

C) Squamous carcinoma cells of SCC4

| **Conc. (**µ**g/ml)** | **Asiatic Acid (% cytotox)** | **Taxol (% cytotox)** |
|---|---|---|
| 50 | 75 | 40 |
| 40 | 70 | 60 |
| 20 | 80 | 60 |
| 10 | 75 | 60 |
| 5 | 75 | 52 |
| 0 | 0 | 0 |

D) SV40-transformed normal epithelial cell line, OKF6

| **Conc. (**µ**g/ml)** | **Asiatic Acid (% cytotox)** | **Taxol (% cytotox)** |
|---|---|---|
| 25 | 92 | 75 |
| 20 | 92 | 48 |
| 10 | 88 | 54 |
| 5 | 23 | 41 |
| 2.5 | 5 | 26 |

E) SK-BR3 breast cancer cell line

| **Conc. (µg/ml)** | **Asiatic Acid (% cytotox)** | **Taxol (% cytotox)** |
|---|---|---|
| 25 | 80 | 50 |
| 12.5 | 23 | 40 |
| 6.2 | 0 | 50 |
| 0.8 | 0 | 40 |

These preliminary data indicate that asiatic acid exerts more potent cytotoxicity to the squamous carcinoma cells of the oral cavity as seen in SCC4 and SCC15 cell lines than the solid tumor cell line of SK-BR3.

HeLa cells were treated with asiatic acid at cytotoxic concentrations and apoptosis of the cells was measured by DAPI staining of the nuclei (2). These results indicate that asiatic acid exerts its cytotoxic effects in HeLa cells by inducing apoptotic cell death.

### References:

(1) Effects of the pH dependence of 3-(4,5 dimethyl-thiazole-2-yl)-2,5-diphenyl-etrazolium bomide-formazan absorption on chemosensitivity determined by in novel tetrazolium-based assay. Jane A. Plumb, R. Milroy and S.B. Kaye in Cancer Res. 49:4435-40, 1989.
(2) Photofootprinting of drug-binding sites on DNA using diazo- and azido-9-aminoacridine derivatives. Claus Jeppesen and Peter E. Nielsen, Eur J. Biochem. 182:437-444, 1989.

## Claims

1. Use of a composition comprising an isolated molecule of asiatic acid or asiaticoside in the manufacture of a medicament for treatment of cancer of epithelial cell origin.

2. The use of claim 1, wherein the treatment either inhibits further growth of cancer or results in regression of cancer.

3. The use of claim 1, wherein the subject is otherwise free of symptoms treatable by asiatic acid or asiaticoside.

4. The use of claim 1, which is for the treatment of cancer in a patient to which a non-asiatic acid, non-asiaticoside anticancer compound is also to be administered.

## Patentansprüche

1. Verwendung einer Zusammensetzung umfassend ein isoliertes Molekül asiatische Säure oder Asiaticosid zur Herstellung eines Medikaments zur Behandlung von Krebs epithelialen Zellursprungs.

2. Verwendung nach Anspruch 1, worin die Behandlung entweder ein weiteres Krebswachstum inhibiert oder zu einem Rückgang von Krebs führt.

3. Verwendung nach Anspruch 1, worin das Subjekt ansonsten frei von Symptomen ist, die mit asiatischer Säure oder Asiaticosid behandelbar sind.

4. Verwendung nach Anspruch 1 zur Behandlung von Krebs bei einem Patienten, dem außerdem eine anti-Krebsverbindung zu verabreichen ist, die keine asiatische Säure und kein Asiaticosid ist.

## Revendications

1. Utilisation d'une composition comprenant une molécule isolée d'acide asiatique ou d'asiaticoside dans la production d'un médicament destiné au traitement du cancer ayant pour origine les cellules épithéliales.

2. Utilisation suivant la revendication 1, dans laquelle le traitement inhibe une croissance supplémentaire du cancer ou bien a pour résultat une régression du cancer.

3. Utilisation suivant la revendication 1, dans laquelle le sujet est par ailleurs dépourvu de symptômes pouvant être traités par l'acide asiatique ou l'asiaticoside.

4. Utilisation suivant la revendication 1, qui est destinée au traitement d'un cancer chez un patient auquel doit également être administré un composé anticancéreux ne consistant pas en acide asiatique ou asiaticoside.
